# EUROPEAN PATENT SPECIFICATION

(11) **EP 1 838 670 B1**
(45) Date of publication and mention of the grant of the patent: **14.10.2009**
(21) Application number: 05815876.7
(22) Date of filing: 05.12.2005
(51) Int. Cl.: C07D 209/88, A61K 31/403, A61P 9/04, A61P 9/12

(54) **PROCESS FOR THE PREPARATION OF CARVEDILOL AND ITS ENANTIOMERS**
VERFAHREN ZUR HERSTELLUNG VON CARVEDILOL UND SEINEN ENANTIOMEREN
PROCEDE DE PREPARATION DE CARVEDILOL ET DE SES ENANTIOMERES

(30) Priority: 09.12.2004 EP 04106438
(43) Date of publication of application: 03.10.2007
(73) Proprietor: ZaCh System S.p.A., 20091 Bresso (Milano) (IT)
(72) Inventor: TREPAT GUIXER, Elisenda, Zambon SA, E-08130 Sta Perpetua de Mogoda (ES); MUNOZ ALVAREZ, Anna, Zambon SA, E-08130 Sta Perpetua de Mogoda (ES); POMARES MARCO, Marta, Zambon SA, E-08130 Sta Perpetua de Mogoda (ES); MARQUILLAS OLONDRIZ, Francisco, Zambon SA, E-08130 Sta Perpetua de Mogoda (ES)
(86) International application number: PCT/EP2005/056469
(87) International publication number: WO 2006/061364

(56) References cited:
- US-A- 4 503 067
- US-A- 4 697 022

## Description

The present invention relates to a process for the preparation of carvedilol as well as of the optically active R and S enantiomers thereof and of mixtures of these enantiomers and, more particularly, relates to an improved process for the preparation of carvedilol and its enantiomers characterized by the use of ethyl acetate as reaction solvent.

Carvedilol, (±)-1-(carbazol-4-yloxy)-3-[[2-(2-methoxyphenoxy)ethyl]amino]-2-propanol, is a nonselective β-adrenergic blocker with α₁-blocking activity. Carvedilol is the active ingredient of COREG^{®} and it is indicated for the treatment of congestive heath failure and for the management of hypertension.

Carvedilol was first described in US 4,503,067 (Boehringer Mannheim GmbH) and the preparation described therein corresponds to the following reaction scheme:

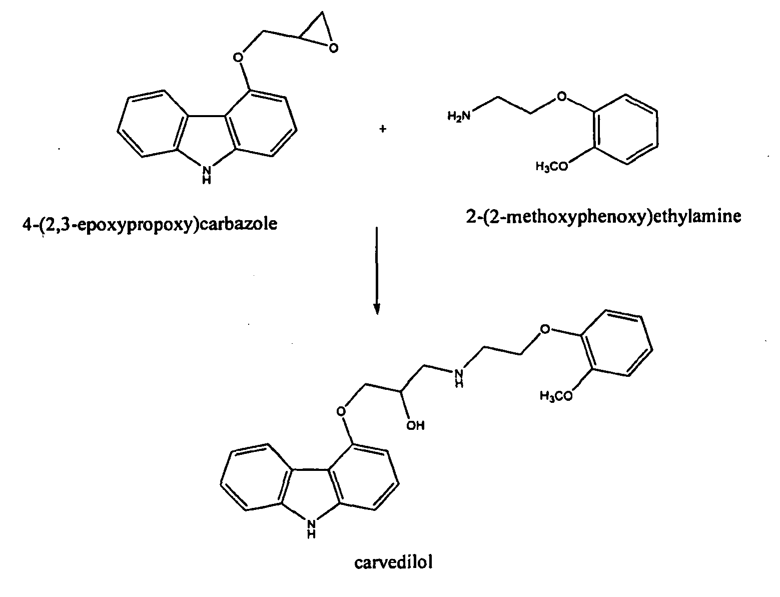

For an easier reference, intermediates 4-(2,3-epoxypropoxy)carbazole and 2-(2-methoxyphenoxy)ethylamine will be indicated herein after also as EPOC and MFA, respectively.

According to US 4,503,067, the reaction between EPOC and MFA is preferably carried out in a solvent which is inert under reaction conditions, for example toluene, dioxan, ethylene glycol dimethyl ether, isopropanol or dimethylformamide.

In example 2 of US 4,503,067, carvedilol is prepared by reaction of EPOC with MFA in ethylene glycol dimethyl ether as reaction solvent. Crude carvedilol is then triturated with diethylether and recrystallized from ethyl acetate to give pure carvedilol form II (m.p. 114°C-115°C).

As described in US 4,697,022, the preparation of carvedilol enantiomers follows the same reaction scheme. In examples 7 and 8 of US 4,697,022, (R) and (S)-carvedilol are prepared from the respective EPOC enantiomers by reaction with MFA in isopropanol.

The process for the preparation of carvedilol or its enantiomers described in US 4,503,067 and US 4,697,022 have some drawbacks, mainly due to the formation of a bis-impurity deriving from the reaction of 2 molar equivalents of EPOC with 1 molar equivalent of MFA.

These drawbacks can be overcome either by using a high excess (higher than 2.8) of MFA, as described in WO02/00216 (Teva Pharmaceutical Industries) or by using a benzyl derivative of MFA (benzyl-MFA), as described in EP 0 918 055 (Egis Gyogyszergyar).

Both alternative methods, however, are not industrially advantageous since they require the use of a high amount of reactants (MFA), which remains unreacted and should be recovered from the reaction mixture, or the addition of a further step (debenzylation) in the process.

We have now found an improved process for the preparation of carvedilol which does not show the drawbacks of the already known processes and allows to prepare carvedilol or its enantiomers in good yields and with high purity.

Therefore, object of the present invention is a process for the preparation of carvedilol or its enantiomers by reaction of EPOC or its enantiomers with an excess of MFA characterized by the fact that the reaction solvent is ethyl acetate.

The improved process object of the present invention has the advantage of requiring no additional step in the synthesis, no high excess of MFA and, moreover, it allows to carry out the overall process by using the same solvent, ethyl acetate, that is the same solvent used also for the final purification/crystallization of the product.

The man skilled in the art can easily acknowledge that the same advantages can derive from the use of other acetic acid esters, such as isopropyl acetate and the like, as a reaction solvent. Exclusively for practical reasons, ethyl acetate is the preferred solvent in the process object of the present invention.

It is evident to the man skilled in the art the advantage deriving from the use of the same solvent in the overall process. However, the replacement of the reaction solvents described in the literature with ethyl acetate is a solution to the problem of the prior art processes which cannot be derived from the prior art teaching.

In fact, US 4,503,067 and US 4,697,022 describe several solvents useful for the reaction of EPOC or its enantiomers with MFA but all these solvents must be inert under reaction conditions. Ethyl acetate is an ester and, as any carbonyl derivative, it cannot be considered an inert solvent in the presence of amines as reactants (for a general reference see Jerry March - Advanced Organic Chemistry - Third Edition, 1985, John Wiley & Sons -page 375 ).

Indeed, in the process object of the present invention some impurities deriving from the use of ethyl acetate can be detected in the reaction mixture as well as in crude carvedilol. These impurities are mainly acetyl-carvedilol and acetyl-MFA of formula

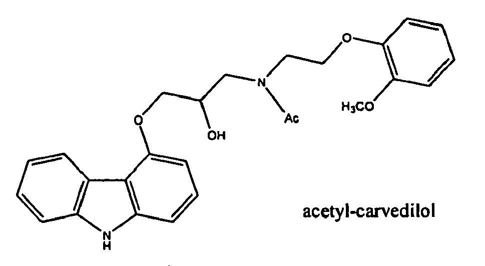

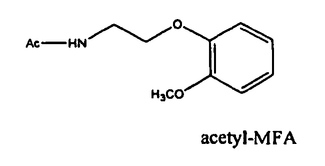

However, the amount of these impurities in the reaction mixture is always lower than 0.5% and they can be easily removed from the final product by crystallization in ethyl acetate according to known methods.

Moreover, ethyl acetate is specifically mentioned to be a useless solvent in this kind of reaction. See in particular EP 0 918 055 which describes that by replacing ethylene glycol dimethyl ether with ethyl acetate in the reaction between EPOC and benzyl-MFA, practically no reaction occurs.

In the process object of the present invention an excess of MFA over EPOC or its enantiomers is used. Preferably the molar excess is from 1.5:1 to 2.5:1, most preferably from 1.8:1 to 2.2:1. Still more preferred molar ratio MFA:EPOC is 2:1.

The reaction between EPOC or its enantiomers and MFA is carried out under heating.

The reaction temperature is preferably from 50°C to the reflux temperature of the reaction mixture. More preferably the reaction is carried out under reflux (about 78°C).

Generally, the reaction takes some hours to be completed depending on the reaction temperature.

The process object of the present invention is preferably used for the preparation of carvedilol, more preferably for the preparation of carvedilol form II.

Crude carvedilol or crude carvedilol enantiomers are separated from the reaction mixture by cooling at 0°C÷-5°C after filtration of the activated carbon eventually added to the reaction mixture.

The resultant crude wet carvedilol or carvedilol enantiomer is then purified by crystallization in ethyl acetate according to known methods.

Carvedilol and its enantiomers are obtained with high yields and high purity.

Carvedilol and carvedilol enantiomers obtained with the process object of the present invention are particularly suitable for the pharmaceutical use.

Therefore, pharmaceutical compositions containing a therapeutically effective amount of carvedilol or an enantiomer thereof prepared according the process of the present invention in admixture with a suitable pharmaceutically acceptable carrier are a further object of the present invention.

Preferred pharmaceutical compositions according to the present invention are tablets, still more preferred are tablets containing carvedilol.

Particularly preferred pharmaceutical compositions are tablets containing carvedilol form II.

The pharmaceutical compositions according to the present invention contains conventional pharmaceutically acceptable carrier and can be prepared according to conventional method.

A practical embodiment of the process object of the present invention is the following.

Ethyl acetate, activated carbon, EPOC and a molar excess of MFA are added into a reactor and the resultant mixture is heated under reflux temperature for about 6 hours.

Then, the activated carbon is filtered off and the resultant solution is cooled to room temperature and then to about 0÷-5°C and kept under stirring.

The crystals are separated by centrifugation and washed with ethyl acetate.

The resultant crude wet carvedilol is dissolved in ethyl acetate by heating under reflux.

After cooling, separation by centrifugation and drying, pure carvedilol form II is obtained.

For better illustrating the invention the following examples are given.

### Example 1

About 4 parts of ethyl acetate were charged into a reactor, under stirring. About 1.4 parts of MFA, about 0.045 parts of activated carbon and about 1 part of EPOC were added.

The mixture was heated to the reflux temperature of ethyl acetate (about 78°C).

The reaction mixture was stirred at about 78°C for about six hours.

The progress of the reaction was checked by TLC.

When the reaction was completed, the mixture was filtered at a temperature not below 65°C in order to separate the activate carbon.

The mixture was cooled to room temperature and then to about 0 ÷ -5°C and stirred for about 1 hour.

The resultant crystals were separated by centrifugation and washed with about 1 part of ethyl acetate.

The resultant wet crude carvedilol was charged into a stainless steel reactor and about 6 parts of ethyl acetate and 0.045 parts of activated carbon were added.

The mixture was heated to the reflux temperature of ethyl acetate (about 78°C), until the dissolution of the crystals. The mixture was stirred at about 78°C for about 1 hour and then filtered at a temperature not below 65°C in order to separate the activate carbon.

The mixture was allowed to cool at about 20°C and then to about 0 ÷ -5°C and stirred for about 1 hour.

The resultant crystals were separated by centrifugation and washed with about 1 part of ethyl acetate.

The wet crystallized carvedilol was charged into a stainless steel reactor and about 4 parts of ethyl acetate were added.

The mixture was heated to the reflux temperature of ethyl acetate (about 78°C), until dissolution of the crystals.

The mixture was stirred at about 78°C for about 1 hour and filtered at a temperature not below 65°C.

The mixture was allowed to cool at about 20°C and then to about 0 ÷ -5°C and stirred for about 1 hour.

The resultant crystals were separated by centrifugation and washed with about 1 part of ethyl acetate.

The wet product was dried in an air dryer at 50°C until the residual solvent ethyl acetate was within the specifications.

Yield: about 1.05 to 1.10 parts of pure carvedilol for 1 part of EPOC.

### Example 2

By repeating the procedure as described in example 1 but carrying out the reaction at a temperature of 70°C, 60°C and 50°C, substantially the same results were obtained with a prolonged reaction time of 8 hours, 10 hours and 16.5 hours, respectively.

### Example 3

The procedure as described in example 1 was repeated obtaining substantially similar results by using a molar ratio EPOC:MFA of 1:1.5, 1:1.7, 1:1.8 and 1:2.2.

## Claims

1. A process for the preparation of carvedilol or its enantiomers by reaction of 4-(2,3-epoxypropoxy)carbazole or its enantiomers with an excess of 2-(2-methoxyphenoxy)ethylamine **characterized by** the fact that the reaction solvent is an acetic acid ester.

2. A process according to claim 1 **characterized by** the fact that the reaction solvent is ethyl acetate.

3. A process according to claim 1 wherein 2-(2-methoxyphenoxy)ethylamine is used in molar excess from 1.5:1 to 2.5:1.

4. A process according to claim 3 wherein the molar excess is from 1.8:1 to 2.2:1.

5. A process according to claim 4 wherein the molar excess is 2:1.

6. A process according to claim 1 for the preparation of carvedilol form II, **characterised by** a melting point of 114 - 115 °C.

## Patentansprüche

1. Verfahren für die Herstellung von Carvedilol oder seiner Enantiomere durch die Reaktion von 4-(2,3-Epoxypropoxy)carbazol oder seiner Enantiomere mit einem Überschuss an 2-(2-Methoxyphenoxy)ethylamin, **dadurch gekennzeichnet, dass** das Reaktionslösungsmittel ein Essigsäureester ist.

2. Verfahren nach Anspruch 1, **dadurch gekennzeichnet, dass** das Reaktionslösungsmittel Ethylacetat ist.

3. Verfahren nach Anspruch 1, wobei 2-(2-Methoxy-phenoxy)ethylamin im molaren Überschuss von 1,5 : 1 bis 2,5 : 1 verwendet wird.

4. Verfahren nach Anspruch 3, wobei der molare Überschuss 1,8 : 1 bis 2,2 : 1 beträgt.

5. Verfahren nach Anspruch 4, wobei der molare Überschuss 2 : 1 beträgt.

6. Verfahren nach Anspruch 1 für die Herstellung von Carvedilol der Form II, **gekennzeichnet durch** einen Schmelzpunkt von 114 - 115°C.

## Revendications

1. Processus de préparation de carvédilol ou de ses énantiomères par réaction de 4-(2,3-époxypropoxy)carbazole ou ses énantiomères avec un excès de 2-(2-méthoxyphénoxy)éthylamine **caractérisé par le fait que** le solvant de réaction est un ester d'acide acétique.

2. Processus selon la revendication 1, **caractérisé par le fait que** le solvant de réaction est de l'acétate d'éthyle.

3. Processus selon la revendication 1, dans lequel 2-(2-méthoxyphénoxy)éthylamine est utilisée dans un excès molaire de 1,5 : 1 à 2,5 : 1.

4. Processus selon la revendication 3, dans lequel l'excès molaire est de 1,8 : 1 à 2,2 : 1.

5. Processus selon la revendication 4, dans lequel l'excès molaire est de 2 : 1.

6. Processus selon la revendication 1 pour la préparation de forme II de carvédilol, **caractérisé par** un point de fusion de 114 - 115° C.
